# EUROPEAN PATENT APPLICATION

(11) **EP 1 302 203 A1**
(43) Date of publication of application: **16.04.2003**
(21) Application number: 01950023.0
(22) Date of filing: 18.07.2001
(51) Int. Cl.: A61K 31/352, C07D 311/36, A61K 35/78, A61P 43/00, A61K 7/06, A61P 15/00

(54) **STEM CELL REINFORCING MATERIAL**

(30) Priority: 18.07.2000 JP 2000217967
(71) Applicant: Nichimo Co., Ltd, Tokyo 140-0002 (JP)
(72) Inventor: TAKEBE, Minoru, Nichimo Co., Ltd., Tokyo 140-0002 (JP); PAN, Weijun, Nichimo Co., Ltd., Tokyo 140-0002 (JP)
(74) Representative: Ouzman, Beverley Nicola Claire
(86) International application number: JP0106246
(87) International publication number: WO02005811

(57) **Abstract**

The stem cell-augmenting material of the present invention being capable of augmenting stem cells consisting of somatic stem cells, particularly hematopoietic stem cells of bone marrow, and embryonic stem cells by being absorbed *in vivo* by oral ingestion, drops, etc. and capable of augmenting stem cells using isoflavone aglycone, which has estrogen-like activity and does not block the enzyme activity of enzymes that act on cell proliferation factor. There have been no stem cell-augmenting materials like the present invention whatsoever. The main characteristic of the present invention is that a stem cell-augmenting material that has the excellent activity is obtained because it has isoflavone aglycone that has a stem cell-augmenting activity.

## Description

### Technical Field

The present invention relates to a stem cell-augmenting material with a function of augmenting stem cells of animals including humans.

### Background Art

Stem cells are generally classified as embryonic stem cells (ES cells) and somatic stem cells. The embryonic stem cells (ES cells) have special properties in that they can be differentiated in all cells that form the body of an animal. The somatic stem cells are present in each organ and tissue of the body of animals and become the basis of the respective organogenesis and histogenesis. Hematopoietic stem cells, nerve stem cells, bone marrow stem cells, liver stem cells, kidney stem cells, etc. are known as the somatic stem cells.

One type of somatic stem cell, hematopoietic stem cells, are stem cells of the blood, and they are the most important stem cells in terms of maintaining hematopoietic function. It is known that the hematopoiesis is performed by bone marrow. It is also known that during gestation, organs that perform hematopoiesis change as the monthly age of the fetus changes, and hematopoietic activity is realized immediately before birth. Hematopoiesis by bone marrow involves first the production of hematopoietic stem cells, and then proliferation and differentiation of these hematopoietic stem cells become erythrocytes and leukocytes that will be supplied *in vivo.* Consequently, the hematopoietic activity of bone marrow of continuous normal production of hematopoietic stem cells is indispensable to maintaining the health of humans and other animals including livestock, etc.

On the other hand, it is said that when large doses of radiation exposure are given or anti-cancer drugs are administered, death does not occur by these acute diseases; but rather, death occurs because there is damage to and a reduction in bone marrow hematopoietic function, that is, the function of producing hematopoietic stem cells, as an adverse effect of the treatments, and thus there is a reduction in the production of blood cells consisting of erythrocytes and leukocytes, etc. as well.

When there is damage to this blood-cell producing function, a problem occurs in that life is interrupted by blood deficiency, even if there is a reduction in or elimination of the cancer cells by the cancer therapy. In other words, cancer can be fought by realizing the effects of cancer therapy as long as the hematopoietic cells of bone marrow are normally maintained, but this is not possible today.

Therefore, there is a need for the development of a material that will augment hematopoietic stem cells of the bone marrow in order to improve the effect of treatment by cancer therapy, etc.

There is further a need for development of a material that will augment hematopoietic stem cells of the bone marrow in order to augment physical strength and to reinforce resistance, even of healthy bodies.

In addition, prevention and treatment of various diseases can be conducted by way of augmenting somatic stem cells other than hematopoietic cells. For instance, Alzheimer's disease, etc. can be prevented and treated by augmenting nerve stem cells; osteoporosis, etc. can be prevented and treated by augmenting bone marrow stem cells; liver disease can be prevented and treated by augmenting liver stem cells; and kidney disease can be prevented and treated by augmenting kidney stem cells.

Furthermore, cell differentiation of embryonic stem cells can be promoted by augmenting embryonic stem cells. There are reports that embryonic stem cells contain STAT3 as a signal-transmitting substance; and this STAT3, which is maintained as is without differentiation of embryonic stem cells, readily reacts with female hormones to initiate embryonic stem cell proliferation. Consequently, selective estrogen receptor modulators (SERMs) such as tamoxifen are being studied as replacements for female hormones. Nevertheless, the mode of action of SERMs has yet to be explained in detail, and there is a demand for SERMs that pose few adverse effects such as toxicity, etc. and are effective in terms of function in humans. From this point, isoflavone aglycone can be expected to act and function efficiently as an SERM.

### Disclosure of Invention

The present invention was made in light of the above points; and the first object of the present invention is to provide a stem cell-augmenting material capable of augmenting stem cells consisting of somatic stem cells, particularly bone marrow hematopoietic stem cells, and embryonic stem cells by being absorbed *in vivo* by oral ingestion, drops, etc.

The second object of the present invention is to provide a stem cell-augmenting material that is capable of augmenting stem cells using isoflavone aglycone, which possesses estrogen-like activity and does not block the enzyme activity of enzymes that act on cell proliferation factor.

The inventors of the present application performed intense research and completed the present invention upon discovering that when rats that had been exposed to radiation orally ingested one or both of the products of promoting proliferation of the lactic acid bacteria contained in the product and/or the lactic acid bacteria that has been added to the product during further hydrolysis of the product of fermentation of isoflavone aglycone and grains by koji mold to decompose the proteins and then received a bone marrow transplant from a healthy rat, significant recovery of spleen function was observed, and therefore, the product of promoting proliferation of the lactic acid bacteria contained in the product and/or the lactic acid bacteria that has been added to the product during further hydrolysis of the product of fermentation of isoflavone aglycone and grains by koji mold to decompose the proteins is absorbed *in vivo*; resulting in that hematopoietic stem cells of the bone marrow can be augmented.

As a result of further intense research, the inventors completed the present invention upon discovering that stem cells are augmented by the activity (estrogen-like activity) of daizein, which is a type of isoflavone aglycone, contained in either of the products of promoting proliferation of lactic acid bacteria contained in the product and/or the lactic acid bacteria that has been added to the product during further hydrolysis of the product of fermenting isoflavone aglycone and grains by koji mold to decompose the proteins.

The stem cell-augmenting activity will be described below. The stem cells augmented by the isoflavone aglycone obtained by the present invention have the properties of self-replication and differentiation and proliferation; and enzymes that act on proliferation factor, such as tyrosine kinase, are related to this cell proliferation. However, because genistein, which is a type of isoflavone aglycone, instead acts to inhibit this tyrosine kinase activity, proliferation of stem cells cannot be expected with genistein. In contrast to this, it appears that the composition of the isoflavone aglycone obtained by the present invention, particularly isoflavone aglycone the components of which have been concentrated by solvent extraction, comprises large amounts of daizein and small amounts of genistein, and therefore, the main function is the estrogen-like activity of the large daizein content, resulting in the augmentation of stem cells.

Furthermore, there are signal-transmitting substances in embryonic stem cells as well as somatic stem cells, and it is clear that STAT3 in particular has a close relationship with cell differentiation. Activation of STAT3 has the effect of maintaining stem cells in an undifferentiated state, but it appears that when the fusion protein (STAT3ER) of STAT3 and the ligand-binding region of estrogen receptor (ER) is formed, undifferentiated colonies are formed, and there is further differentiation and proliferation. Therefore, the daizein that is a type of isoflavone aglycone obtained by the present invention has an estrogen-like role and apparently binds with ER to actuate STAT3ER activity and proliferate bone marrow hematopoietic cells. As a result, embryonic stem cells wherein STAT3 is the signal-transmitting substance can also be augmented by the isoflavone aglycone obtained by the present invention.

The stem cell-augmenting material of the present invention obtained as described above is characterized in that it contains isoflavone aglycone that has stem cell-augmenting activity. These stem cells are embryonic stem cells or somatic stem cells that include stem cells that become the basis of the respective organogenesis and histogenesis, including hematopoietic stem cells, nerve stem cells and bone marrow stem cells. Estrogen-like activity that does not block the enzyme activity of enzymes that act on cell proliferation factor can be given as the activity of this isoflavone aglycone in terms of augmenting stem cells.

When the stem cell-augmenting material containing isoflavone aglycone that has the stem cell-augmenting activity of the present invention is absorbed in vivo, somatic stem cells, on the one hand, can be enhanced.

When the stem cell-augmenting material that contains isoflavone aglycone that has the stem cell-augmenting activity of the present invention is actuated on a signal-transmitting substance of embryonic stem cells, these embryonic stem cells, on the other hand, can be augmented.

Looking next at hematopoietic stem cells specifically, hematopoietic stem cells of bone marrow can be augmented by *in vivo* absorption of the stem cell-augmenting material that contains isoflavone aglycone which has stem cell-augmenting activity.

More specifically, the isoflavone aglycone includes daizein that possesses estrogen-like activity as a function of augmenting hematopoietic stem cells of bone marrow. Consequently, when the isoflavone aglycone is absorbed *in vivo,* the daizein has the same role as estrogen, and it binds with the ER of hematopoietic stem cells so as to actuate the activity of STAT3ER and augments the hematopoietic activity of bone marrow. Thus, hematopoietic cells proliferate and differentiate to become erythrocytes and leukocytes; and blood having the optimum level of components, such as blood cells, etc. is supplied *in vivo*.

Thus, when the stem cell-augmenting material of the present invention is absorbed *in vivo* by oral ingestion, drops, etc., there is constant normal production of hematopoietic stem cells since the hematopoietic effect of bone marrow and blood having the optimum level of components, such as blood cells, etc., is supplied *in vivo.* Therefore, a reduction in hematopoietic function by bone marrow that occurs when, for instance, radiation or anti-cancer drugs are given for cancer therapy is prevented; and there is strong recovery of the hematopoietic function that has been diminished. As a result, it can be expected that cancer therapy can be done without adverse reaction so as overcome cancer by way of absorbing the stem cell-augmenting material of the present invention *in vivo* by oral ingestion, drops, etc..

When the stem cell-augmenting material of the present invention is absorbed *in vivo* by oral ingestion, drops, etc., augmentation of hematopoietic stem cells of the bone marrow is realized; and the function of supplying blood that has the optimum level of components, such as blood cells, etc., *in vivo* in order to prevent disease and maintain health can be further expected.

In addition, when the isoflavone aglycone of the present invention is absorbed *in vivo,* somatic stem cells other than these hematopoietic stem cells are likewise augmented.

Furthermore, for embryonic stem cells, daizein plays a role similar to estrogen in terms of a signal- transmitting substance of embryonic stem cells, STAT3, and it binds with ER to form an STAT3ER with the ligand-binding region of ER. The STAT3ER promotes undifferentiated colony formation and differentiation and proliferation as well.

Isoflavone aglycone that possesses the above described activity is preferably a material derived from grains. Moreover, pulse crops are particularly preferred as the grain-derived materials. It is preferred that the isoflavone aglycone be produced by performing fermentation of pulse crops by koji mold and then performing hydrolysis on the components of pulse crops by the enzymes of koji mold. It is further preferred that the isoflavone aglycone obtained as described above be produced by extraction and concentration by a solvent. In this case, the isoflavone aglycone should contain at least 70 wt% daizein.

The stem cell-augmenting material of the present invention thus produced as described above becomes a product that has a high isoflavone aglycone content by thorough decomposition of the proteins and/or carbohydrates contained in soybean meal as a result of koji preparation treatment, in which pulse crops are fermented by koji mold, and then hydrolysis; and the augmenting activity of somatic stem cells, including the above-described bone marrow hematopoietic stem cells, and embryonic stem cells can be more efficiently conducted.

Furthermore, .the stem cell-augmenting material of the present invention is characterized in that it is a product of promoting proliferation of lactic acid bacteria contained in the product and/ or lactic acid bacteria added to the product during further hydrolysis of the product of fermentation of pulse crops by koji mold so as to decompose the proteins (called, "koji mold soybean fermentation and cultivation product" hereafter).

This koji mold soybean fermentation and cultivation product has the same function as the above-described isoflavone aglycone to augmente somatic stem cells, including bone marrow hematopoietic stem cells, and embryonic stem cells, and it realizes the same excellent activity as the isoflavone aglycone.

### Brief Description of Drawings

Figure 1 is a diagram of the processes that produce the stem cell-augmenting material of the present invention,
Figure 2 is a graph showing changes in spleen weight after radiation exposure in Example 1 in rats, etc., that had ingested the stem cell-augmenting material of the present invention,
Figure 3 is a graph showing changes in spleen weight after radiation exposure per 100 g body weight in Example 1 of rats, etc., that had ingested the stem cell-augmenting material of the present invention,
Figure 4 is a graph showing the number of spleen colonies that formed following marrow transplant after radiation exposure in Example 1 of rats, etc., that had ingested the stem cell-augmenting material of the present invention,
Figure 5 is a graph showing changes in body weight in Example 1 of rats, etc., that had ingested the steam cell-augmenting material of the present invention,
Figure 6 is a graph showing changes in spleen weight after radiation exposure per 10 g body weight in Example 2 of rats, etc., that had ingested the stem cell-augmenting material of the present invention,
Figure 7 is a graph showing the number of spleen colonies that formed following bone marrow transplant after radiation exposure in Example 2 of rats, etc., that had ingested the stem cell-augmenting material of the present invention,
Figure 8 is a graph showing changes in testicle weight after radiation exposure per 10 g body weight in Example 2 of rats, etc., that had ingested the stem cell-augmenting material of the present invention,
Figure 9 is a graph showing changes in body weight in Example 2 of rats, etc., that had ingested the stem-cell augmenting material of the present invention,
Figure 10 is a graph showing changes in spleen weight after radiation exposure per 10 g body weight in Example 3 of rats, etc., that had ingested the stem cell-augmenting material of the present invention,
Figure 11 is a graph showing the number of spleen colonies that formed following bone marrow transplant after radiation exposure in Example 3 of rats, etc., that had ingested the stem cell-augmenting material of the present invention,
Figure 12 is a graph showing changes in testicle weight after radiation exposure per 10 g body weight in Example 3 of rats, etc., that had ingested the stem cell-augmenting material of the present invention,
Figure 13 is a graph showing changes in body weight in Example 3 of rats, etc., that had ingested the stem-cell augmenting material of the present invention,
Figure 14 is a graph showing changes in spleen weight after radiation exposure per 10 g body weight in Example 4 of rats, etc., that had ingested the stem cell-augmenting material of the present invention,
Figure 15 is a graph showing the number of spleen colonies that formed following bone marrow transplant after radiation exposure in Example 4 of rats, etc., that had ingested the stem cell-augmenting material of the present invention, and
Figure 16 is a graph showing changes in body weight in Example 4 of rats, etc., that had ingested the stem cell-augmenting material of the present invention.

### Best Mode for Carrying Out the Invention

Embodiments of the present invention will be described below.

The stem cell-augmenting material, that includes hematopoietic stem cell-augmenting material, of the present invention, that augments stem cells consisting of somatic stem cells and embryonic stem cells, is characterized in that it has isoflavone aglycone and/or koji mold soybean fermentation and cultivation product. More specifically, the stem cell-augmenting material, including hematopoietic stem cell-augmenting material, of the present invention is used as a solid (including all forms, such as powder, granules, etc.) or liquid food or drug that contains isoflavone aglycone and/or koji mold soybean fermentation and cultivation product which has a stem cell-augmenting activity.

The isoflavone aglycone can be obtained from any starting material; and it is preferred that the isoflavone aglycone be a material derived from cereals obtained from grains used as the starting material. Moreover, it is particularly preferred that the grain-derived material be produced by fermentation of pulse crops by koji mold and then by hydrolysis so as to decompose the components of the pulse crops by enzymes of the koji mold. It is further preferred that the material produced by the hydrolysis be further extracted and concentrated by a solvent so to produce isoflavone aglycone.

When the stem cell-augmenting material of the present invention that contains isoflavone aglycone which is obtained as described above is continuously absorbed *in* *vivo* for a specific time by oral administration or drops, somatic stem cells, including bone marrow hematopoietic stem cells, can be augmented.

In addition, when the stem cell-augmenting material of the present invention that contains isoflavone aglycone having the stem cell-augmenting activity is actuated on a signal-transmitting substance of embryonic stem cells, the embryonic stem cells can be augmented.

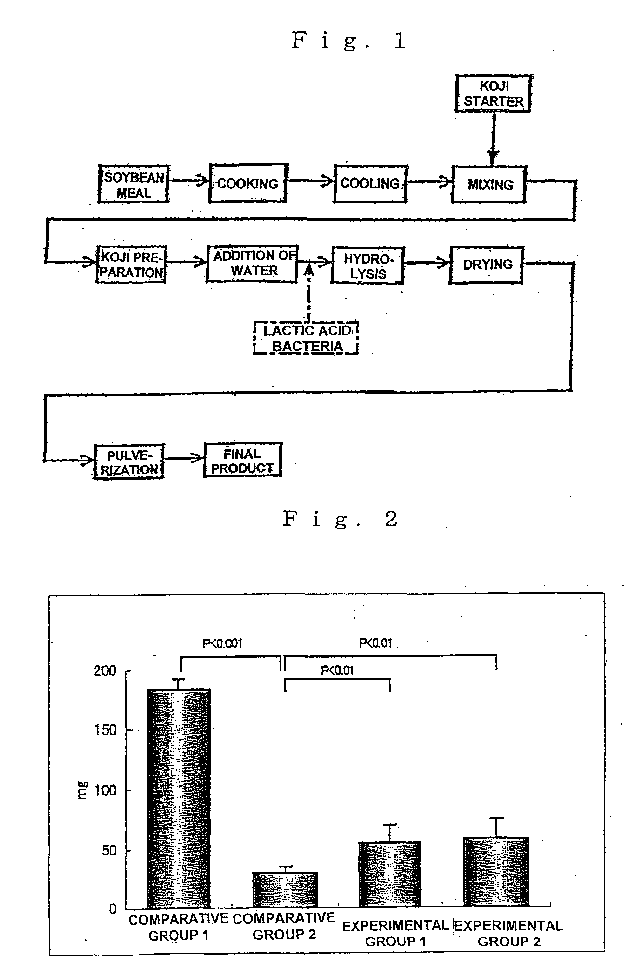

The manner of producing, by way of fermentation using koji mold, the stem cell-augmenting material, including the hematopoietic stem cell-augmenting material, of the present invention, which contains isoflavone aglycone and augments stem cells including somatic stem cells and embryonic stem cells, will be described next with reference to Figure 1.

Figure 1 shows, with the solid lines therein, one embodiment of the production method whereby the stem cell-augmenting material of the present invention is produced from soybean meal, which is a type of pulse crop; and it further shows, with the broken lines, an embodiment of the method of producing the koji mold soybean fermentation and cultivation product.

The manner of producing the stem cell-augmenting material of the present invention by the processes shown by the solid lines in Figure 1 will be described first. First, the soybean meal is cooked. Propagation of the soybean meal is facilitated by this cooking when compared to when cooking is not performed. Depending on the purpose of production, etc, cooking of the soybean meal is performed by the batch method, whereby cooking and subsequent koji preparation are separately performed, or by the continuous method using a koji preparation device with which cooking and subsequent koji preparation are continuously performed.

Once cooking has been completed, the water content of the soybean meal is adjusted to the content with which propagation of koji mold can be accomplished (for instance, approximately 36 wt%).

The stem cell-augmenting material of the present invention is produced in the following manner from soybean meal whose water content has been adjusted in this way.

More specifically, when the cooked soybean meal simple substance is cooled to bring its temperature to 40°C or lower so as not to kill the koji mold, the soybean meal is inoculated with koji starter consisting of koji mold to a specific weight ratio, and then these two are mixed until a uniform mixture is obtained.

So as to raise the temperature of the mixture as a result of propagation of the koji starter, the temperature of the mixture is once reduced inside a koji preparation device and the mixture is set aside while keeping temperature at approximately 32°C. As a result, the soybean meal is fermented by the koji mold, and temperature rises to approximately 38°C as koji preparation thus proceeds; and the product becomes firm and solidifies as hyphae of the propagating koji mold grow. Then, as koji preparation proceeds, the solidified product is released from the koji preparation device by being spun and agitated. Ventilation is provided in order to uniformly feed air to the inside and control temperature so that product temperature is reduced to approximately 33 - 35°C. Koji preparation proceeds further as ventilation is continued. As a result, death of the koji mold is reliably prevented, and propagation of koji mold is satisfactorily accomplished. Then, the product inside the koji preparation device is spun and agitated enough times depending on how firmly the product has solidified inside the koji preparation device as ventilation is continued.

The koji mold used for the above koji preparation is the koji mold that has been used for many years in fermented food products unique to Japan and "tempe." The genus *Aspergillus,* including *Aspergillus usamii, Aspergillus kawachii, Aspergillus awamori*, *Aspergillus saitoi, Aspergillus oryzae, Aspergillus niger,* etc., and those classified as the genus *Rhizops,* which are safe for food products, are preferably used.

The fermentation time is at least 24 hours or longer, depending on the species of koji mold that is used. The fermentation time should be long enough to thoroughly propagate the koji and decompose a specific amount of proteins and/or saccharides in the soybean meal.

In this embodiment, hydrolysis of the proteins and/or saccharides is further performed on the product of koji preparation. Here, the term saccharides includes saccharides such as sucrose, etc. and starches etc.

Hydrolysis after this koji preparation treatment is also performed while keeping the substrate as a flake state. In other words, in this embodiment, water is added to the product after completing koji preparation to bring the water content to, for instance, 50 wt%, and then the product (resultant) is heated to 30 - 45°C, and temperature is kept constant for a specific time in order to hydrolyze the proteins and/or carbohydrates contained in the product of koji preparation treatment. The proteins and/or carbohydrates are thus hydrolyzed, and large amounts of isoflavone aglycone that has the function of augmenting hematopoietic stem cells of the bone marrow are produced.

Next, specific examples of the stem cell-augmenting material, including hematopoietic stem cell-augmenting material, of the present invention that augments somatic stem cells and embryonic stem cells will be described below.

The fermented soybean meal, which becomes the stem cell-augmenting material that is produced by the present invention using *Aspergillus oryzae* as the koji mold as described above, is soybean meal in which the isoflavone compounds have been thoroughly decomposed. In other words, although untreated soybean meal contains relatively large amounts of daizin and genistin, which are glycosides, when compared to daizein and genistein, which are aglycones, the fermented soybean meal produced by the present invention contains very little daizin and genistin, that is glycosides, because they have been decomposed, but contains a very large amount of daizein and genistein, that is aglycones, which are produced by decomposition. Table 1 shows the isoflavone compound content of untreated soybean meal obtained by adjusting product temperature to 35°C while ventilating, performing koji preparation for 48 hours, adding water to a water content of approximately 50%, and then setting aside for 24 hours or longer at 45°C to allow the soybean components and koji mold components to be hydrolyzed by the enzymes in the koji mold. It is clear that large amounts of daizein and genistein, which are the aglycone form of isoflavone compounds, are obtained.

**Table 1**

| Daizin | Daizein | Genistin | Genistein |
|---|---|---|---|
| Not detected | 70 | 103 | 64 |
| (Units: mg/100 g) | | | |

Soybean embryo will be described below. After soybean embryo was pre-steamed and then water was added to bring the water content to approximately 37%, the resultant was steamed, sterilized, and cooled; and then with respect to the mixture ratio of soybean embryo and koji mold, 200 g inoculum that had been adjusted to 8 x 10⁷ koji mold spores/g (adjusted with polished rice) were mixed per 400 kg soybean embryo. Furthermore, after cooling the resultant to 32°C when koji preparation was started, koji preparation proceeded without ventilation until the product temperature reached 40°C; and when temperature reached 40°C, the koji preparation proceeded with an increase in temperature being prevented while providing ventilation. The first agitation ("mori" process) was conducted for approximately 17 hours after starting of koji preparation. The soybean embryo gave off heat, and temperature was controlled while providing ventilation so that the product temperature after agitation would be approximately 35°C. Next, the second agitation ("naka" process) was performed after approximately eight hours, and heat was given off. The product temperature was again controlled to approximately 35°C by ventilation, and the third agitation ("shimai" process) was further performed as in the previous process after approximately 16 hours. Then, koji preparation was completed, approximately 48 hours after starting, by controlling temperature while providing ventilation so that the product temperature would be approximately 38°C. Once koji preparation was completed, water content of the product was adjusted while agitating it to bring the water content to 50%, and the product was heated to a temperature of approximately 50°C. Then, the majority of the isoflavone compounds in the soybean embryo were hydrolyzed to aglycone form by the koji mold enzymes for 48 hours or longer. Isoflavone compounds of the soybean embryo obtained by treatment of the present invention were obtained in large quantities, mainly in the aglycone form of daizein, as shown in Table 2.

**Table 2**

| Components | | Present invention | Untreated |
|---|---|---|---|
| Glycoside | Daizin | 70 | 840 |
| | Genistin | 40 | 170 |
| | Glycitin | 130 | 620 |
| | Total | 240 | 1630 |
| Aglycone | Daizein | 680 | 10 |
| | Genistein | 130 | 2.4 |
| | Glycitein | 240 | 2.0 |
| | Total | 1050 | 14.4 |

Moreover, it is preferred that the product obtained by hydrolysis be further extracted and concentrated using solvent in the present embodiment to obtain a concentrated product of 30 wt% or more isoflavone aglycone as shown in Table 3. The product includes approximately 70 wt% daizein as isoflavone aglycone in this case.

**Table 3**

| Composition of Concentrated Product of Material of the Present Invention | | |
|---|---|---|
| Analysis items | Standard value | Analytical value |
| Properties | Pale yellow powder with no offensive taste, offensive odor or impurities | Passed |
| Weight loss on drying | 5% or less | 2.8% |
| Ignition residue | 3% or less | 0.1% |
| Arsenic | 2 ppm or less | Passed |
| Heavy metals | 20 ppm or less | Passed |
| Agricultural chemicals residue | Not detected | Not detected |
| General number of live bacteria | 3000 bacteria/g or less | 3000 bacteria/g or less |
| *Escherichia coli* group | Negative | Negative |
| Amount of isoflavone aglycone | 30% or more | 32.9% |
| Daizein | | 23.4% |
| Glycitein | | 8.0% |
| Genistein | | 1.5% |

The stem cell-augmenting material of the present embodiment that is produced as described above is a product that contains a high isoflavone aglycone content as a result of thorough decomposition of proteins and carbohydrates contained in soybean meal by koji preparation treatment and subsequent hydrolysis.

This isoflavone aglycone has the function of augmenting somatic stem cells, such as the function of being capable of augmenting bone marrow hematopoietic stem cell; and when it is absorbed *in vivo,* bone marrow hematopoietic activity, for instance, is augmented, and hematopoietic stem cells proliferate and differentiate to become erythrocytes and leukocytes, and blood having the optimum level of components, such as blood cells, etc. is supplied *in vivo*.

Thus, when the stem cell-augmenting material of the present invention is absorbed *in vivo* by oral ingestion, drops, etc., there is constant normal production of hematopoietic stem cells as the hematopoietic effect of bone marrow and blood having the optimum level of components, such as blood cells, etc., is supplied *in vivo.* Therefore, for instance, a reduction in hematopoietic function by bone marrow that occurs when large dose of radiation exposure are given or radiation or anti-cancer drugs are given for cancer therapy is prevented, and there is strong recovery of the hematopoietic function that has been diminished. As a result, it can be expected to be able to give cancer therapy without adverse reaction and thereby overcome cancer by absorbing the stem cell-augmenting material of the present invention *in vivo* by way of oral ingestion, drops, etc..

In addition, when the stem cell-augmenting material of the present invention is absorbed in *vivo* by oral ingestion, drops, etc.., augmentation of hematopoietic stem cells of the bone marrow to realize the function of supplying blood having the optimum level of components, such as blood cells, etc., in *vivo* in order to prevent disease and maintain health can be further expected. Effective maintenance of health can be expected with ingestion of the stem cell-augmenting material of the present invention particularly by persons working in nuclear facilities and persons working with radioactivity, such as X-rays, etc.

Consequently, when the stem cell-augmenting material containing isoflavone aglycone of the present invention that is formed as described above is continuously absorbed *in vivo* for a specific time by oral administration or drops, somatic stem cells, including bone marrow hematopoietic stem cells, can be augmented.

In addition, by way of actuating the stem cell-augmenting material, containing isoflavone aglycone that has a stem cell-augmenting activity of the present invention, on the signal-transmitting substance of embryonic stem cells, these embryonic stem cells can be augmented.

Next, the method of producing the koji mold soybean fermentation and cultivation product, which is the stem cell-augmenting material of the present invention, will be described with reference to the broken lines in Figure 1.

This koji mold soybean fermentation and cultivation product is a product of promoting proliferation of lactic acid bacteria contained in the above-described product and/or lactic acid bacteria added to the product during further hydrolysis of the product of fermentation of pulse crops by koji mold to decompose the proteins. It differs from the method for producing the above-described isoflavone aglycone in that lactic acid bacteria are also used during hydrolysis.

In the present embodiment, koji mold and lactic acid bacteria are allowed to coexist so as to promote their proliferation during hydrolysis of the product produced by koji preparation treatment, thus obtaining koji mold soybean fermentation and cultivation product.

There are two methods of using this lactic acid bacteria.

In the first embodiment of one method that uses lactic acid bacteria, product is produced by hydrolysis for the necessary time until the number of lactic acid bacteria reaches 10⁸ cfu/g or more. Other process in this method are performed as in the above-described embodiment. More specifically, in this embodiment, lactic acid bacteria naturally contained in the product of koji preparation treatment is used. In addition, with the use of probiotics, lactic acid and koji mold actively proliferate together by coexisting and living on the same substrate made from the product from pulse crops, which are the starting material.

The lactic acid mold was separated from the fermented soybean meal produced in accordance with the production method of the present invention using as the koji mold *Aspergillus oryzae,* which is the koji mold for miso, and the lactic acid bacteria were identified by conducting tests on the test items shown in Table 4.

**Table 4**

| Properties of Isolated Bacteria a | |
|---|---|
| Test item | Test results |
| Morphology | Coccus *1 |
| Gram staining | + *1 |
| Spores | - *1 |
| Motility | - *1 |
| Behavior in oxygen | Facultative anaerobic *1 |
| Catalase | - *1 |
| Lactic acid product | L(+) *1 |
| Production of gas from glucose | - *1 |
| Viable at 10°C | + |
| Viable at 45°C | + |
| Viable at 50°C | + |
| Viable in the presence of 6.5% NaCl | + |
| Viable at pH of 9.6 | + |
| Viable in the presence of 40% bile | + |
| Production of yellow pigment | - |
| Hemolytic (ovine blood) | α hemolysis |
| Arginine dihydrolase | + |
| Hydrolysis of equine uric acid | + |
| Hydrolysis of esculin | + |
| Viable in the presence of 0.1% methylene blue milk | + |
| Production of acid | |
| D-xylose | - |
| L-ramnose | + *2, *3 |
| Sucrose | + |
| Lactose | + |
| Melibiose | + |
| Raffinose | + *2 |
| Melezitose | - |
| Glycerol | - *2, *3 |
| Adonitol | - |
| Sorbitol | + *2 |
| Mannitol | + |
| L-arabinose | + |
| GC content of DNA in cell walls of bacterium (mol%) *4 | 40 |

| | |
|---|---|
| *¹Shows the results of previous report (Test Report No. 598-010212-001). | |
| *²Atypical property. | |
| *³However, *Enterococcus faesium* JCM5804^{T} (reference strain) showed the same reaction as isolated bacterium a. | |
| *⁴ By HPLC. | |

Based on the properties in Table 4, the isolated lactic acid bacteria was identified to be *Enterococcus faesium* of the genus *Enterococcus.* This lactic acid bacteria was therefore confirmed to be an intestinal flora called enterococcus.

The above-described fermentation time and hydrolysis time as well as hydrolysis temperature in the present example should be adjusted in accordance with the type, state, properties, and quantity of pulse crop; type, state, properties and quantity of koji mold; type, state, properties, and quantity of lactic acid bacteria; and type, properties, etc., of the stem cell-augmenting material, which is the product.

Next, the second embodiment of the method that uses lactic acid to product koji mold soybean fermentation and cultivation product will be described with reference to the broken lines in Figure 1.

In this embodiment, lactic acid bacteria are added to the product of koji preparation treatment during hydrolysis.

This lactic acid bacteria is preferably the same *Enterococcus faesium* of the genus *Enterococcus* as in the above-described first embodiment.

When the lactic acid bacteria has been introduced to the product of koji preparation treatment as described above and hydrolysis is started, the koji mold that have proliferated during koji preparation treatment and lactic acid bacteria coexist in the above-described product, or the same substrate. As a result, the lactic acid bacteria receives nutrients from the product to promote its proliferation during hydrolysis, and the koji mold and lactic acid bacteria are cultivated together in the product. In particular, vitamin Bs are produced in the product by koji preparation treatment. Since components that are easily absorbed and are of high nutritional value to the lactic acid bacteria are produced, proliferation of the lactic acid bacteria that uses these components as a nutrient source is promoted. Furthermore, hydrolysis of various components by enzymes of the koji mold corresponds to decomposition of the soybean meal by digestive enzymes and the digested product and undigested product are separated in the product of the present embodiment. The separated product (digested product) that has been hydrolyzed by the enzyme of koji mold is directly absorbed when it is ingested by humans and livestock, but the undecomposed product becomes a useful nutrient source for lactic acid bacteria that are always present within the intestines without being absorbed and increases the production of short-chain fatty acids that are useful to humans and livestock.

Moreover, the above-described product is a culture medium in which lactic acid bacteria can actively proliferate. Accordingly, the lactic acid bacteria that have proliferated can always be present within the intestines of humans and livestock by way of using enzyme undecomposed product contained in the above-described product as the nutrient source. As a result, the stem cell-augmenting material is eventually produced from a pulse crop starting material that possesses the ability to promote proliferation of lactic acid bacteria by using probiotics, wherein whereby koji mold and useful microorganisms are cultivated in a product that serves as the same substrate.

The stem cell-augmenting material of the present embodiment produced as described above has the excellent function, as in the isoflavone aglycone of the above-described embodiment, to augment stem cells consisting of somatic stem cells, including bone marrow hematopoietic stem cells, and embryonic stem cells.

Next, examples of the present invention will be described.

### Example 1

The mouse spleen colony method accompanied by exposure to radiation and bone marrow transplant was performed on rats in order to conduct experiments confirming the function of augmenting bone marrow hematopoietic stem cells in the present example.

### Experimental method

1) Healthy female rats of equal body weight in each group were reared as the sample (female ICR) (eight weeks old).
2) Ordinary food, feed of isoflavone agylone concentrated material mixed with this ordinary food, and feed of the product of promoting proliferation with koji mold and lactic acid both present during further hydrolysis of the product produced by koji preparation treatment of pulse crop starting material as koji mold soybean fermentation and cultivation product mixed with the ordinary food, were used as the substances administered to the rats.
3) The rats were divided into comparative groups 1 and 2 and experimental groups 1 and 2 of five rats each. As shown in Table 5, ordinary food was administered to comparative groups 1 and 2, while ordinary food to which isoflavone aglycone had been added at a ratio of 30 mg/kg body weight was administered to experimental group 1, and ordinary food to which 1 wt% of the koji mold soybean fermentation and cultivation product had been added was administered to experimental group 2. Each group was reared for three weeks.

**Table 5**

| Administration group | | | Administration substance | Dose (mg/kg) | Number of animals |
|---|---|---|---|---|---|
| Comparative group 1 | Radiation unexposed | Bone marrow transplanted | Ordinary food | -- | 5 |
| Comparative group 2 | Radiation exposed | Bone marrow transplanted | Ordinary food | -- | 5 |
| Experimental group 1 | Radiation exposed | Bone marrow transplanted | Ordinary food + concentrated material (0.1%) | Isoflavone aglycone 30 mg/kg | 5 |
| Experimental group 2 | Radiation exposed | Bone marrow transplanted | Ordinary food + koji mold soybean fermentation and cultivation product (1%) | -- | 5 |
| *The (mg/kg) in the table are per body weight. | | | | | |

4) Radiation exposure
The rats of comparative group 2 and experimental groups 1 and 2 were systemically exposed one time only to a dose of 8 Gy after three weeks of rearing.
5) Bone marrow transplant
Bone marrow transplant was performed on the rats of each group 24 hours after radiation exposure. In this case, the femur of a donor rat of the same species was cut out. A small piece was cut off from both ends of the femur with a clean bench. Once 1 ml RPMI medium 1640 medium containing 10% antibiotic-antimycotic was injected with a syringe from one end through the medullary cavity, it was pushed into a test tube. The marrow cell count was adjusted to 10⁵/ml, and 0.5 ml was injected from a caudal vein. The donor rat had been systemically exposed to 2 Gy radiation and reared for four weeks on feed of 1 wt% of the koji mold soybean fermentation and cultivation product added to ordinary food.
6) Determination of spleen weight
All rats were sacrificed, the spleen was excised, and spleen weight and the number of spleen colonies that had formed were determined after determining body weight on day 7 after bone marrow transplant.
7) Determination of body weight
Body weight of all rats was determined before bone marrow transplant and on day 7 after transplant.

### Experimental results

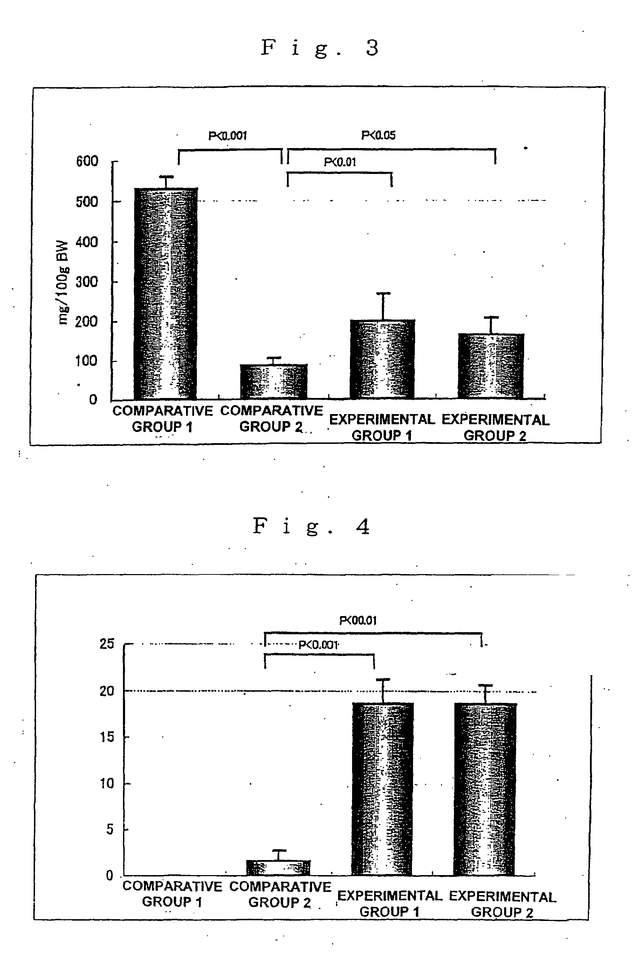

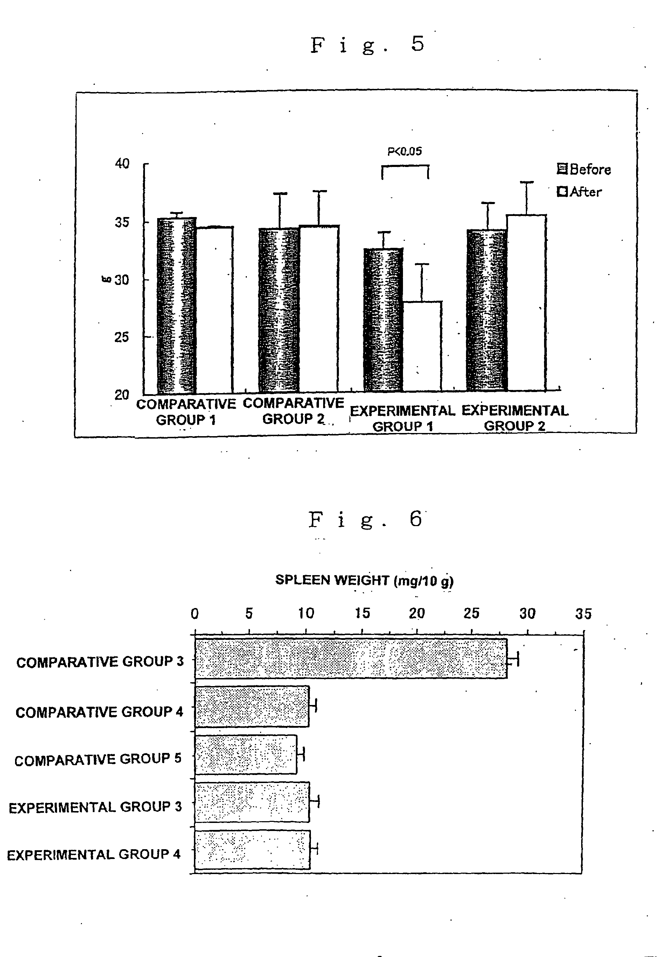

1) Changes in spleen weight after radiation exposure are as shown in Figure 2, changes in spleen weight after radiation exposure per 100 g body weight are as shown in Figure 3, and the number of spleen colonies that formed following bone marrow transplant after radiation exposure is as shown in Figure 4.
2) The results of determining body weight are as shown in Figure 5.

According to the experimental results pertaining to the spleen shown in Figures 2 through 4, when the other comparative group 2 and experimental groups 1 and 2 are seen in comparison to rats that did not receive radiation exposure in comparative group 1, the increase in spleen weight was small and the number of spleen colonies that formed was also low in comparative group 2 that did not ingest the stem cell-augmenting material of the present invention; thus, bone marrow hematopoietic function is markedly diminished. According to the results of comparative group 2, when there was radiation exposure at a strong dose of 8 Gy, almost no effect was realized with bone marrow transplant, and the number of spleen colonies that formed was kept very low in the rats that were given ordinary food only.

In contrast to this, there was a large increase in spleen weight and the number of spleen colonies that formed was also extremely high in experimental group 1 and experimental group 2 that ingested the stem cell-augmenting material of the present invention. It is, therefore, clear that there is significant improvement of the function of augmenting bone marrow hematopoietic stem cells.

More specifically, in contrast to comparative group 2 that was given ordinary food only, the effect of receiving a bone marrow transplant was immediately realized, and the number of spleen colonies that formed was extremely high, even when there was radiation exposure to a strong dose of 8 Gy, when the stem cell-augmenting material containing isoflavone aglycone and koji mold soybean fermentation and cultivation product of the present invention was absorbed *in vivo.* Consequently, remarkable recovery of bone marrow hematopoietic function that was diminished by radiation exposure was possible by *in vivo* absorption of the stem cell-augmenting material containing isoflavone aglycone and koji mold soybean fermentation and cultivation product of the present invention. Thus, for instance, when there has been radiation exposure to a weak dose of 6 Gy, 4 Gy, etc., the same high number of spleen colonies that have formed and remarkable recovery of bone marrow hematopoietic function that was diminished by the radiation exposure as obtained by bone marrow transplant can be expected if the stem cell-augmenting material containing isoflavone aglycone as well as the koji mold soybean fermentation and cultivation product of the present invention is absorbed *in vivo* without performing bone marrow transplant.

Bone marrow hematopoietic function is safely recovered as seen from the above; therefore, it is clear that the stem cell-augmenting material of the present invention has the function of augmenting bone marrow hematopoietic stem cells.

Consequently, it is clear that when the stem cell-augmenting material consisting of isoflavone aglycone as well as the koji mold soybean fermentation and cultivation product is absorbed *in vivo,* bone marrow hematopoietic stem cells can be augmented. As a result, hematopoietic stem cells will proliferate and differentiate to become erythrocytes and leukocytes and blood having the optimum level of components, such as blood cells, etc., will be supplied *in vivo*.

Thus, when the stem cell-augmenting material of the present invention is absorbed *in vivo* by oral ingestion, drops, etc., there is constant normal production of hematopoietic stem cells as the hematopoietic effect of bone marrow and blood having the optimum level of components, such as blood cells, etc., is supplied *in vivo.* Therefore, for instance, the reduction in hematopoietic function by bone marrow, that occurs when large dose of radiation exposure are given or radiation or anti-cancer drugs are given for cancer therapy, is prevented; and there is strong recovery of the hematopoietic function that has been diminished. As a result, it can be expect to be able to give cancer therapy without adverse reaction and thereby overcome cancer by absorbing the stem cell-augmenting material of the present invention *in vivo* by oral ingestion, drops, etc..

In addition, when the stem cell-augmenting material of the present invention is absorbed *in vivo* by oral ingestion, drops, etc.., augmentation of bone marrow hematopoietic stem cells is realized, so that the function of supplying blood having the optimum level of components, such as blood cells, etc., *in vivo* in order to prevent disease and maintain health can further be expected.

### Example 2

In order to conduct experiments for confirming the function of augmenting bone marrow hematopoietic cells in male recipients, the function of augmenting reproductive stem cells, and dependency on the dose of isoflavone aglycone administered, the mouse spleen colony method accompanied by radiation exposure and bone marrow transplant was performed using rats.

### Experimental method

1) Healthy male rats of equal body weight in each group were reared as the sample (male ICR) (six weeks old). They were reared with 10 rats each in comparative groups 3, 4 and 5 as well as experimental group 4 and 5 rats in experimental group 3.
2) Ordinary food, feed of isoflavone aglycone concentrated material mixed with this ordinary food, and feed of toremifen, which is a selective estrogen receptor modulator (SERM) and is a derivative of lower toxicity than tamoxifen, mixed with the ordinary food, were used as the substances administered to the rats.
3) As shown in Table 6, ordinary food was administered to both comparative groups 3 and 4. Experimental group 3 was reared on ordinary food to which isoflavone aglycone concentrated product had been added at a ratio of 30 mg/kg body weight, experimental group 4 was reared on ordinary food to which isoflavone aglycone concentrated material had been added at a ratio of 100 mg/kg body weight, and comparative group 5 was reared on ordinary food to which toremifen had been added at a ratio of 30 mg/kg body weight.

**Table 6**

| Administration group | | | Administration substance | Dose (mg/kg) | Number of animals |
|---|---|---|---|---|---|
| Comparative group 3 | Radiation unexposed | Bone marrow transplanted | Ordinary food | -- | 10 |
| Comparative group 4 | Radiation exposed | Bone marrow transplanted | Ordinary food | -- | 10 |
| Comparative group 5 | Radiation exposed | Bone marrow transplanted | Ordinary food + toremifen (30mg/Kg) | -- | 10 |
| Experimental group 3 | Radiation exposed | Bone marrow transplanted | Ordinary food + concentrated material (0.1%) | Isoflavone aglycone 30 mg/kg | 5 |
| Experimental group 4 | Radiation exposed | Bone marrow transplanted | Ordinary food + concentrated material (0.35%) | Isoflavone aglycone 100 mg/kg | 10 |
| *The (mg/kg) in the table are per body weight. | | | | | |

4) Radiation exposure
The rats of comparative groups 4 and 5 as well as experimental groups 3 and 4 were systemically exposed one time only to a dose of 8 Gy after three weeks of rearing.
5) Bone marrow transplant
Bone marrow transplant was performed on the rats of each group 24 hours after radiation exposure. In this case, the femur of a donor rat of the same species was cut out. A small piece was cut off from both ends of the femur with a clean bench. Once 1 ml RPMI medium 1640 medium containing 10% antibiotic-antimycotic was injected with a syringe from one end through the medullary cavity, it was pushed into a test tube. The marrow cell count was adjusted to 10⁵/ml and 0.5 ml was injected from a caudal vein. The donor rat had been systemically exposed to 2 Gy radiation and reared for three weeks on feed of 1 wt% of the koji mold soybean fermentation and cultivation product added to ordinary food.
6) Determination of spleen and testicle weights
All of the rats were sacrificed, the spleen was excised, spleen weight and the number of spleen colonies formed were determined, and the ovaries were likewise excised and testicle weight was determined after determining body weight on day 7 after bone marrow transplant.
7) Determination of body weight
Body weight of all rats was determined before bone marrow transplant and on day 7 after transplant.

### Experimental results

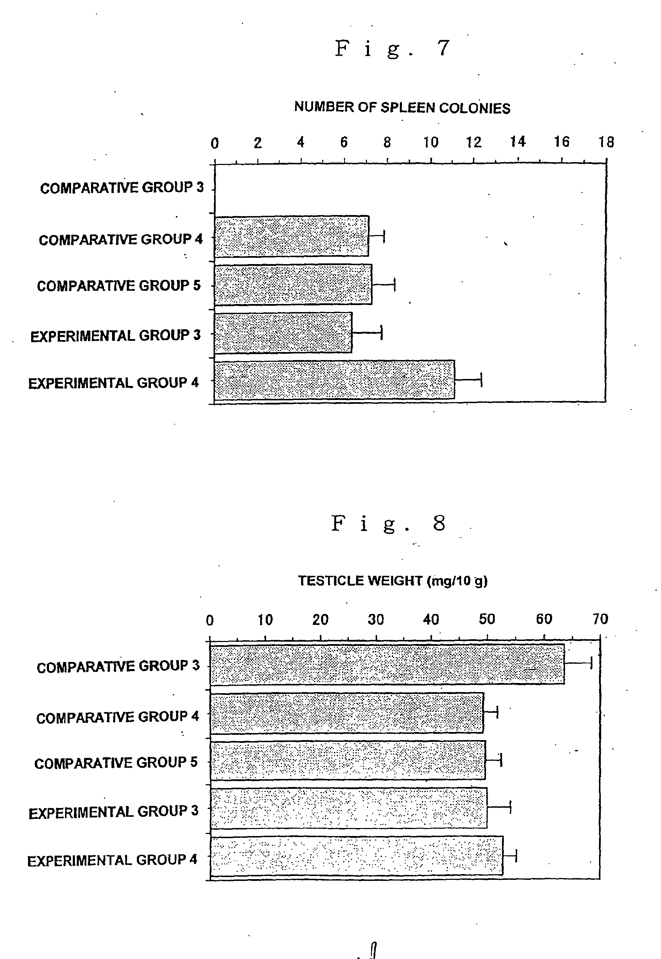

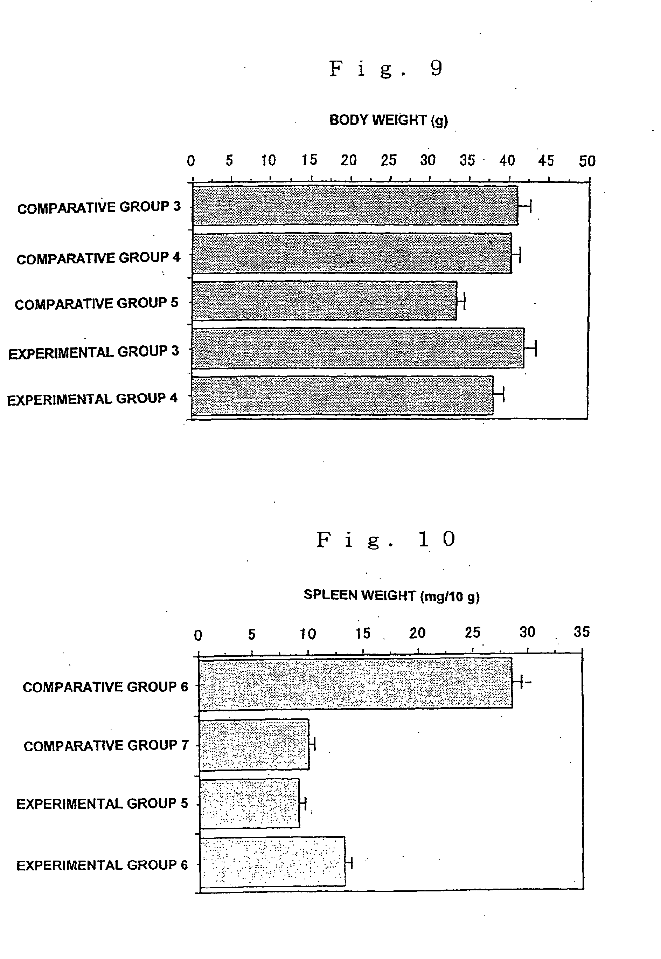

1) Changes in spleen weight after radiation exposure per 10 g body weight are as shown in Figure 6, and the number of spleen colonies that formed following bone marrow transplant after radiation exposure is as shown in Figure 7.
2) Changes in testicle weight after radiation exposure per 10 g body weight are as shown in Figure 8.
3) The results of determining body weight are as shown in Figure 9.

According to the experimental results relating to the spleen of male rats shown in Figures 6 and 7, when the other comparative groups 4 and 5 as well as experimental groups 3 and 4 are seen in comparison to male rats that did not receive radiation exposure in comparative group 3, spleen weight is similarly lower in each of the other groups when compared to comparative group 3; and of these, comparative group 5 shows a somewhat lower value than the others. The number of spleen colonies that formed is significantly higher in experimental group 4, which was administered a ratio of isoflavone aglycone of the stem cell-augmenting material of the present invention to body weight of 100 mg/kg, than the other comparative groups 4 and 5 and experimental group 3. Testicle weight was likewise lower in each of the other groups when compared to comparative group 3; but experimental group 4, which was administered a ratio of isoflavone aglycone in the stem cell-augmenting material of the present invention to body weight of 100 mg/kg, was almost significantly higher than in the other comparative groups 4 and 5 and experimental group 3. Body weight hardly changed in comparative groups 4 and 5 and experimental groups 3 and 4 when compared to comparative group 3, but comparative group 5 showed a significantly lower value than the others.

These experimental results show that although the number of spleen colonies that formed and testicle weight of one of the experimental groups, experimental group 4, which was administered a ratio of isoflavone aglycone of the stem cell-augmenting materials of the present invention to body weight of 100 mg/kg, was significantly augmented when compared to the others, augmenting effects to the extent of showing significance in comparison to the comparative groups was not obtained with the other experimental group 3, which was administered at a ratio of isoflavone aglycone to body weight of 30 mg/kg. Thus, it was shown that there is dose dependency to the amount ingested in order to realize the steam cell-augmenting activity of the stem cell-augmenting material of the present invention, that is, stem cell activity increases with an increase in the dose ingested. Furthermore, it was shown that the same stem cell augmenting-activity as with the female rats in Example 1 is safely realized by ingesting a sufficient amount of the stem cell-augmenting material of the present invention.

Moreover, it is clear that spleen weight and body weight of comparative group 5 are lower than those of the other comparative group 4 and experimental groups 3 and 4. Thus, although it is thought that the toremifen administered to comparative group 5 has the same stem cell-augmenting activity as isoflavone aglycone, it also was considerably stronger than isoflavone aglycone in terms of toxicity. In contrast to this, it was made clear that even if isoflavone aglycone of the stem cell-augmenting material of the present invention is administered at 100 mg/kg per body weight, its toxicity in terms of inhibition of body weight is weaker than that of toremifen; therefore, isoflavone aglycone effectively participates in augmentation of stem cells. In other words, it was clarified that in terms of safety, isoflavone aglycone is effective, even if the amount ingested is increased to augment stem cell-augmenting activity.

Furthermore, testicle weight was almost significantly higher in experimental group 4, which was administered a ratio of the isoflavone aglycone of the stem cell-augmenting material of the present invention to body weight of 100 mg/kg, than in the other comparative groups 4 and 5 and experimental group 3. Accordingly, it was clarified that the isoflavone aglycone of the stem cell-augmenting material of the present invention has augmenting activity on stem cells related to reproductive organs consisting of testes and female ovaries, as well as reproductive cells consisting of sperm and ova.

### Example 3

Male rats were exposed to radiation in order to conduct experiments to confirm the function of augmenting bone marrow hematopoietic cells in male donors, the function of augmenting reproductive stem cells, and dependency relating to the dose of isoflavone aglycone.

### Experimental method

1) Healthy male rats of equal body weight in each group were reared as the sample (male ICR) (six weeks old). Ten animals were reared in each of comparative groups 6 and 7 as well as experimental groups 5 and 6.
2) Normal feed and feed of isoflavone aglycone concentrated material mixed with this normal feed were used as the substances administered to the rats.
3) As seen from Table 7, comparative groups 6 and 7 were administered normal feed, experimental group 5 was reared on feed of isoflavone aglycone concentrated material added to normal feed to a ratio per body weight of 30 mg/kg, and experimental group 6 was reared on feed of isoflavone aglycone concentrated material added to normal feed to a ratio per body weight of 100 mg/kg.

**Table 7**

| Administration group | | | Administration substance | Dose (mg/kg) | Number of animals |
|---|---|---|---|---|---|
| Comparative group 6 | Radiation unexposed | Bone marrow transplanted | Ordinary food | -- | 10 |
| Comparative group 7 | Radiation exposed | Bone marrow transplanted | Ordinary food | -- | 10 |
| Experimental group 5 | Radiation exposed | Bone marrow transplanted | Ordinary food + concentrated material (0.1%) | Isoflavone aglycone 30 mg/kg | 10 |
| Experimental group 6 | Radiation exposed | Bone marrow transplanted | Ordinary food + concentrated material (0.35%) | Isoflavone aglycone 100 mg/kg | 10 |
| *The (mg/kg) in the table are per body weight. | | | | | |

4) Radiation exposure
The rats of comparative group 7 and experimental groups 5 and 6 were systemically exposed one time only to a dose of 2 Gy and reared for three weeks.
5) Determination of spleen weight
All of the rats were sacrificed, the spleen was excised and spleen weight and the number of spleen colonies formed were determined, and the ovaries were similarly excised and testicle weight was determined after determining body weight after rearing for three weeks.
6) Determination of body weight
Body weight of all rats was determined before rearing and after three weeks of rearing.

### Experimental results

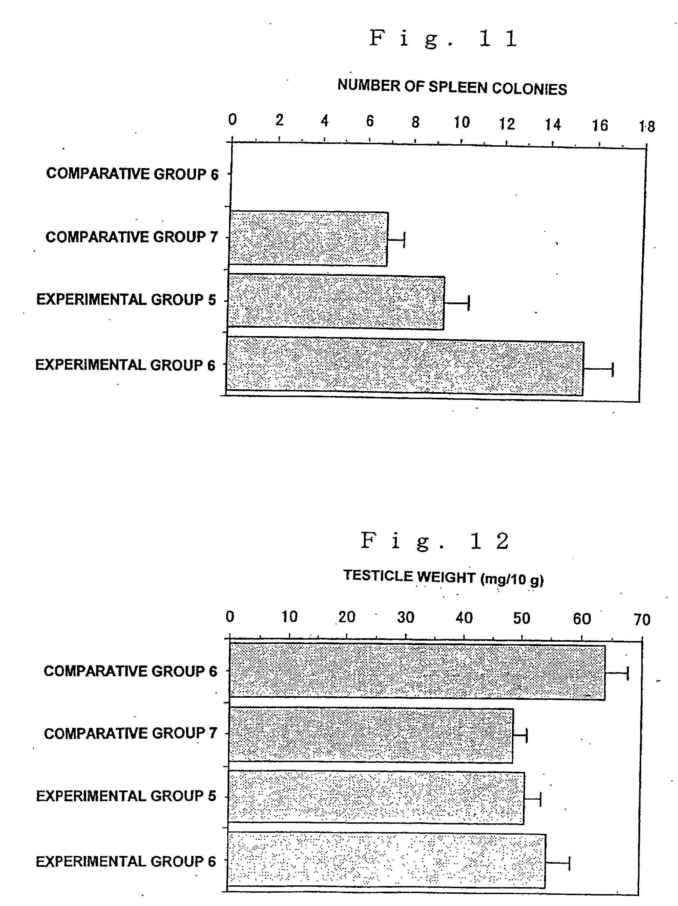

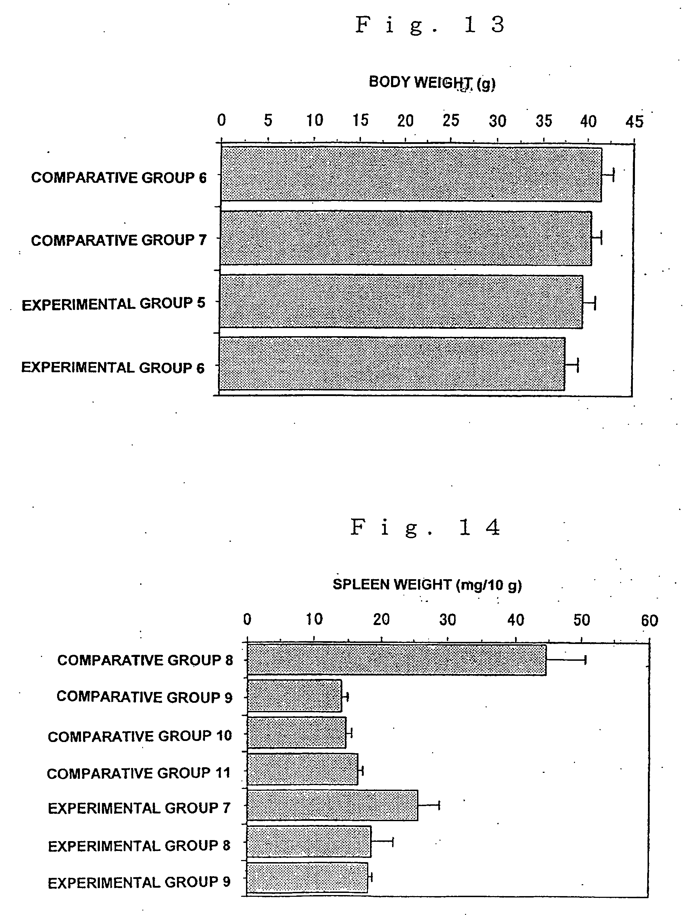

1) Changes in spleen weight after radiation exposure per 10 g body weight are as shown in Figure 10, and the number of spleen colonies formed following bone marrow transplant after radiation exposure is as shown in Figure 11.
2) Changes in testicle weight after radiation exposure per 10 g body weight are as shown in Figure 12.
3) The results of determining body weight are as shown in Figure 13.

Based on the experimental results pertaining to the spleen of male rats shown in Figures 10 and 11, when the other comparative group 7 as well as experimental groups 5 and 6 are seen in comparison to the male rats that did not receive radiation exposure in comparative group 6, spleen weight was similarly low in each of the other groups when compared to comparative group 6; but experimental group 6, which was administered a ratio of isoflavone aglycone of the stem cell-augmenting material of the present invention to body weight of 100 mg/kg, was significantly higher than comparative group 7 and the other experimental group 5. The number of spleen colonies that formed was significantly higher in experimental group 6, which was administered a ratio of isoflavone aglycone of the stem cell-augmenting material of the present invention to body weight of 100 mg/kg, than in comparative group 7; while experimental group 5, which was administered a ratio of isoflavone aglycone of stem cell-augmenting material of the present invention to body weight of 30 mg/kg, was higher than comparative group 7, but not significantly. Testicle weight was likewise lower in each of the other groups when compared to comparative group 6; but experimental group 6, which was administered a ratio of isoflavone aglycone of the stem cell-augmenting material of the present invention to body weight of 100 mg/kg, showed a tendency toward being higher than the other comparative group 7 and experimental group 5. Body weight was somewhat lower in comparative group 7 and experimental groups 5 and 6 when compared to comparative group 6.

According to these experimental results, in male rats that serve as donors, one of the experimental groups, experimental group 6, which was administered a ratio of isoflavone aglycone of the stem cell-augmenting material of the present invention to body weight of 100 mg/kg, showed significant augmentation in terms of the number of spleen colonies formed and testicle weight when compared to the others; but results were obtained showing that although the other experimental group 5, which was administered a ratio of isoflavone aglycone to body weight of 30 mg/kg, was lower than experimental group 6, it was augmented in comparison to comparative group 7. This clarified the fact that in male rats that serve as donors, there is dose dependency to the amount ingested in order to realize stem cell-augmenting activity of the stem cell-augmenting material of the present invention, that is, there is an increase in stem cell activity with an increase in the dose ingested. Furthermore, it was made clear that the same stem cell-augmenting activity as in the female rats in Example 1 can be safely realized by ingesting a sufficient dose of the stem cell-augmenting material of the present invention.

Furthermore, experimental group 6, which was administered a ratio of isoflavone aglycone of the stem cell-augmenting material of the present invention to body weight of 100 mg/kg, showed a tendency toward a higher testicle weight than the other comparative group 7 and experimental group 5. Therefore, it was clarified that the isoflavone aglycone of the stem cell-augmenting material of the present invention has augmenting activity on stem cells related to reproductive organs consisting of testes and female ovaries, as well as reproductive cells consisting of sperm and ova.

Based on these Examples 2 and 3, when isoflavone aglycone, which is the stem cell-augmenting material of the present invention, is ingested by both recipient and donor, there is *in vivo* augmentation of stem cells due to stem cell-augmenting activity, and transplant results are marked when a recipient receives a bone marrow, etc., transplant from a donor, and the donor can provide bone marrow, etc., with better transplant effects.

### Example 4

The mouse spleen colony method accompanied by radiation exposure and bone marrow transplant was conducted in rats in order to perform experiments confirming the function of augmenting bone marrow hematopoietic stem cells, dependency of the dose of isoflavone aglycone, and the difference in function of isoflavone aglycone mixtures as well as singular isoflavone aglycone and function of estrogen having the highest activity.

### Experimental method

1) Healthy female rats of equal body weight in each group were reared as the sample (female ICR) (six weeks old). They were reared with five rats in each group.
2) Ordinary food, and feed of isoflavone aglycone concentrated material mixed with this ordinary food, and feed of genistein as a singular isoflavone aglycone or 17β estradiole, which has the highest estrogen activity, mixed with the ordinary food, were used as the substances administered to the rats.
3) As shown in Table 8, ordinary food was administered to each of comparative groups 8, 9 and 10. Experimental group 7 was reared on ordinary food to which isoflavone aglycone concentrated product had been added at a ratio of 100 mg/kg body weight, experimental group 8 was reared on ordinary food to which isoflavone aglycone concentrated material had been added at a ratio of 30 mg/kg body weight, experimental group 9 was reared on ordinary food to which genistein had been added as singular isoflavone aglycone at a ratio of 30 mg/kg body weight, and comparative group 11 was reared on ordinary food to which 17β estradiole had been added at a ratio of 10 µg/kg body weight.

**Table 8**

| Administration group | | | Administration substance | Dose (mg/kg) | Number of animals |
|---|---|---|---|---|---|
| Comparative group 8 | Radiation unexposed | No bone marrow transplanted | Ordinary food | -- | 5 |
| Comparative group 9 | Radiation unexposed | Bone marrow transplanted | Ordinary food | -- | 5 |
| Comparative group 10 | Radiation exposed | Bone marrow transplanted | Ordinary food | -- | 5 |
| Comparative group 11 | Radiation exposed | Bone marrow transplanted | Ordinary food + 17β estradiole (10 µg/kg) | -- | 5 |
| Experimental group 7 | Radiation exposed | Bone marrow transplanted | Ordinary food + concentrated material (0.35%) | Isoflavone aglycone 100 mg/kg | 5 |
| Experimental group 8 | Radiation exposed | Bone marrow transplanted | Ordinary food + concentrated material (0.1%) | Isoflavone aglycone 30 mg/kg | 5 |
| Experimental group 9 | Radiation exposed | Bone marrow transplanted | Ordinary food + genistein (30 mg/kg) | Genistein 30 mg/Kg | 5 |
| *The (mg/kg) and (µg/kg) in the table are per body weight. | | | | | |

4) Radiation exposure
With the exception of comparative group 8, the rats of comparative groups 9, 10 and 11 as well as experimental groups 7, 8 and 9 were systemically exposed one time only to a dose of 8 Gy after 3 weeks of rearing.
5) Bone marrow transplant
With the exception of comparative groups 8 and 9, bone marrow transplant was performed 24 hours after radiation exposure on the rats of comparative groups 10 and 11 as well as those of experimental groups 7, 8, and 9. In this case, the femur of a donor rat of the same species was cut out. A small piece was cut off from both ends of the femur with a clean bench. Once 1 ml RPMI medium 1640 medium containing 10% antibiotic-antimycotic was injected with a syringe from one end through the medullary cavity, it was pushed into a test tube. The marrow cell count was adjusted to 10⁵/ml and 0.5 ml was injected from a caudal vein. The donor rat had been systemically exposed to 2 Gy radiation and reared for three weeks on feed of 1 wt% of the koji mold soybean fermentation and cultivation product added to ordinary food.
6) Determination of spleen weight
All of the rats were sacrificed, the spleen was excised and spleen weight and the number of spleen colonies formed were determined on day 10 after bone marrow transplant.
7) Determination of body weight
Body weight of all rats was determined when rearing was started, after one week, and after two weeks and before bone marrow transplant and on day 5, day 7 and day 10 after transplant.

### Experimental Results

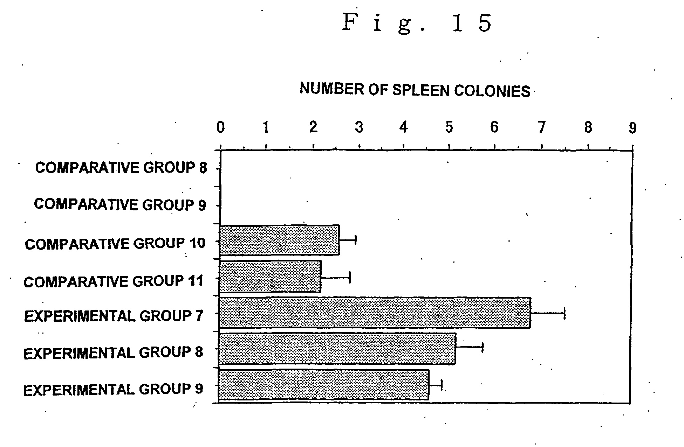

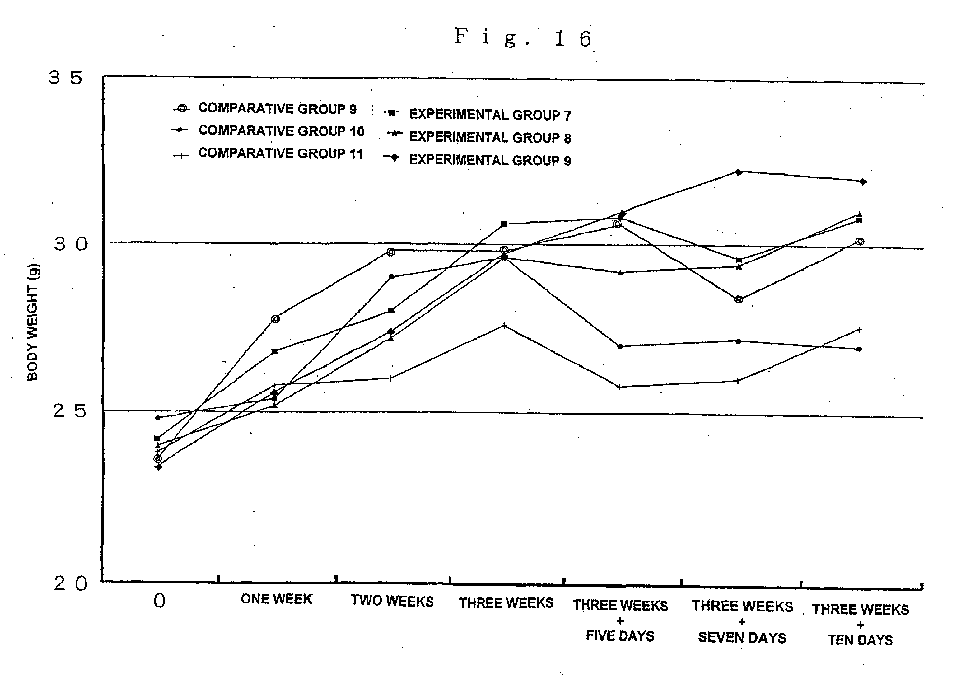

1) Changes in spleen weight after radiation exposure per 10 g body weight are as shown in Figure 14, and the number of spleen colonies that formed following transplant after radiation exposure is as shown in Figure 15.
2) The results of determining body weight are as shown in Figure 16.

Based on the experimental results pertaining to the spleen of female rats in Figures 14 and 15, when the other comparative groups 9, 10 and 11 as well as experimental groups 7, 8 and 9 are seen in comparison to female rats that did not receive radiation exposure in comparative group 8, spleen weight is similarly lower in the other groups when compared to comparative example 8; and of these, comparative groups 9, 10 and 11 show lower values than experimental groups 7, 8 and 9. On the other hand, spleen weight is significantly higher in experimental groups 7, 8 and 9 than in comparative groups 9 and 10, which received radiation exposure; and of these, experimental group 7, which was administered a ratio of isoflavone aglycone of the stem cell-augmenting material of the present invention to body weight of 100 mg/kg, was significantly higher than in the other experimental groups 8 and 9. With respect to the number of spleen colonies that formed, spleen weight was significantly higher in experimental groups 7, 8 and 9 than in comparative groups 10 and 11, which received a transplant after radiation exposure; and of these, experimental groups 7 and 8, which were administered a ratio of isoflavone aglycone of the stem cell-augmenting material of the present invention to body weight of 100 mg/kg and 30 mg/kg, were significantly higher than the other experimental group 9, which was administered a ratio of genistein as singular isoflavone aglycone of 30 mg/kg. Judging from day 7 after radiation exposure, body weight showed a tendency toward a reduction in comparative groups 9, 10 and 11, showed a tendency toward not changing in experimental groups 7 and 8, and showed a tendency toward an increase in experimental group 9.

Based on these results, it is clear that in rats that serve as recipients, experimental groups 7, 8 and 9, which were administered the mixture of isoflavone aglycone of the stem cell-augmenting material of the present invention and singular isoflavone aglycone, were significantly augmented when compared to comparative groups 10 and 11, which received a transplant after radiation exposure, in terms of spleen weight and the number of spleen colonies that formed. Furthermore, it is also clear that in experimental groups 7, 8 and 9, which was administered the stem cell-augmenting material of the present invention, experimental groups 7 and 8, which were administered isoflavone aglycone mixture, were significantly higher than experimental group 9, which was administered singular isoflavone aglycone in terms of spleen weight and the number of spleen colonies that formed, and that in experimental groups 7 and 8, which were administered the stem cell-augmenting material of the present invention, experimental group 7, which was administered a high dose of mixed isoflavone aglycone, was significantly higher than experimental group 8, which was administered less. Thus, it was clarified that in female rat recipients as well, there is dose dependency to the amount of isoflavone aglycone ingested in order to realize the stem cell-augmenting activity of the stem cell-augmenting material of the present invention, that is, there is an increase in stem cell activity with an increase in the dose ingested. Furthermore, it was made clear that the mixture of isoflavone aglycones in Table 3 realizes stronger stem cell-augmenting activity than singular genistein. In particular, the cost of producing the mixture of isoflavone aglycone in Table 3 is less than that of singular genistein; therefore, in terms of cost, the mixture is superior. Moreover, there was marked inhibition of an increase in body weight with 17β estradiole, which has the strongest estrogen activity, and there was no augmentation whatsoever in terms of spleen colony formation. The dose was set at 1/1,000 or less that of isoflavone aglycone in the present experiment, but there was no proof whatsoever of augmentation of stem cells; and instead, adverse reaction, such as inhibition of body weight, etc., became a problem.

The present invention is not limited to the above-described embodiments or examples, and changes can be made as needed. In addition to oral administration as in the above-described examples, the stem cell-augmenting material of the present invention can be absorbed *in vivo* through drops.

## Claims

1. A stem cell-augmenting material, **characterized in that** said stem cell-augmenting material contains isoflavone aglycone that augments stem cells.

2. The stem cell-augmenting material according to claim 1, **characterized in that** said stem cells are somatic stem cells or embryonic stem cells, said somatic stem cells including stem cells that become a basis of various organogenesis and histogenesis, that includes hematopoietic stem cells, nerve stem cells, bone marrow stem cells, and the like.

3. The stem cell-augmenting material according to claim 1 or 2, **characterized in that** said isoflavone aglycone possesses estrogen-like activity and is prevented from blocking enzyme activity of enzymes that act on cell proliferation factor.

4. The stem cell-augmenting material according to any one of claims 1 through 3, **characterized in that** said isoflavone aglycone is a material derived from grains.

5. The stem cell-augmenting material according to claim 4, **characterized in that** a material derived from said grains is produced by performing fermentation on grains by koji mold to decompose proteins thereof and then performing hydrolysis.

6. The stem cell-augmenting material according to claim 5, **characterized in that** said grains are pulse crops.

7. The stem cell-augmenting material according to claim 5 or 6, **characterized in that** said material produced by hydrolysis of isoflavone aglycone is further concentrated.

8. The material having sexual performance-improving activity according to claim 7, **characterized in that** said isoflavone aglycone is comprised of at least 70 wt% daizein.

9. A stem-cell augmenting material, **characterized in that** said stem-cell augmenting material is a product of promoting proliferation of lactic acid bacteria contained in said product and/or lactic acid added to said product during further hydrolysis of said product that is obtained by way of fermentation of pulse crops by koji mold to decompose proteins thereof.
